# EUROPÄISCHE PATENTSCHRIFT

(11) **EP 0 684 012 B1**
(45) Veröffentlichungstag und Bekanntmachung des Hinweises auf die Patenterteilung: **01.12.1999**
(21) Anmeldenummer: 95102142.7
(22) Anmeldetag: 16.02.1995
(51) Int. Cl.: A61B 17/04, A61B 17/06, A61B 17/064, A61B 17/068

(54) **Faden zum Anlegen einer chirurgischen Naht, Kombination aus einem Übergabehalter und dem Faden und Kombination aus einem chirurgischen Nähinstrument und dem Faden**
Thread for applying a surgical suture, combination of a transfer holder and the thread and combination of a surgical suturing instrument and the thread
Fil pour suture chirurgicale, combinaison d'un support de transfert et du fil et combinaison d'un instrument chirurgical pour suture et du fil

(30) Priorität: 28.05.1994 DE 4418766
(43) Veröffentlichungstag der Anmeldung: 29.11.1995
(73) Patentinhaber: Forschungszentrum Karlsruhe GmbH, 76133 Karlsruhe (DE)
(72) Erfinder: Konrad, Joachim, D-76646 Bruchsal (DE); Kuntz, Bertram, D-76889 Steinfeld (DE); Grünhagen, Armin, D-76344 Eggenstein-Leopoldshafen (DE); Schülken, Heinrich, D-76297 Stutensee (DE)
(74) Vertreter: Rückert, Friedrich, Dr.

(56) Entgegenhaltungen:
- EP-A- 0 185 453
- AU-B- 625 522
- FR-A- 2 322 578
- US-A- 4 732 151
- US-A- 4 890 613

## Beschreibung

Die Erfindung betrifft einen Faden zum Anlegen einer chirurgischen Naht gemäß dem Oberbegriff von Anspruch 1, eine Kombination aus einem Übergabehalter und dem Faden gemäß Anspruch 4 und eine Kombination aus einem chirurgischen Nähinstrument und dem Faden gemäß Anspruch 5.

Ein Clip mit den im Oberbegriff von Anspruch 1 genannten Merkmalen ist aus der AU 0 625 522 B bekannt. Bei diesem Clip ist ein biegsames Mittelteil an seinen Enden mit jeweils einem Verschlußteil versehen. Eines der Verschlußteile ist rohrförmig und nimmt das andere Verschlußteil auf. Das andere Verschlußteil besteht aus einem mit einer kegelförmigen Spitze versehenen Zylinder. Da das rohrförmige Verschlußteil einen inneren Absatz aufweist und die Spitze des anderen Verschlußteils an ihrer Basis einen größeren Durchmesser aufweist als der Zylinder, lassen sich die beiden Verschlußteile fest miteinander verriegeln.

Ein Verschlußunterteil für einen Clip mit einem Hohlzylinder, in dessen durchgehende Bohrung temporär während der Applikation eine chirurgische Nadel eingesetzt werden kann, ist aus der FR 2 322 578 A bekannt.

Ein chirurgisches Nähinstrument gemäß den Merkmalen a)-c) spruch 5 ist aus der DE 42 17 202 A1 bekannt. Das Nähinstrument besteht aus einem Griffstück auf der proximalen Seite, zwei Backen, von denen eine aufklappbar ist, auf der distalen Seite, sowie einem Instrumentenschaft, der von einem äußeren Rohr umgeben ist. In Arbeitsstellung liegen die Backen parallel zueinander. Ihre einander gegenüberliegenden Seiten enthalten jeweils mehrere im Abstand zueinander angebrachte Nuten, deren Grundflächen auf einer Schraubenlinie liegen. Durch diese Nuten wird das Nähgut beim Vernähen der Wundränder schraubenlinienartig geformt. Als Nähgut dient ein mechanisch stabiler Draht, mit dem das Gewebe direkt durchstochen wird; das Instrument weist daher keine Nadel auf.

Aus der DE 41 24 381 C1 ist ein chirurgisches Nähinstrument bekannt, dessen Nähkopf aus zwei gleichgeordneten, gleichartig senkrecht zur Nähinstrumentenachse stehenden Fingern besteht, zwischen denen eine Nähnadel umgesetzt werden kann. Der Nähvorgang erfolgt über koaxiale Rohre, so daß das Instrument in der minimalinvasiven Chirurgie einsetzbar ist. Zur Herstellung der Naht wird ein üblicher Faden eingesetzt. Das Verknoten üblicher Fäden ist zeitaufwendig und erfordert einen zur Anlage des Knotens ausreichenden freien Raum im Operationssitus.

Ein ähnliches chirurgisches Nähinstrument, bei dem die Nadel während des Nähens ebenfalls umgesetzt wird, ist in der DE 41 24 383 C1 beschrieben.

Eine besondere Ausführungsform der beiden letztgenannten Nähinstrumente, bei der die Nadel während des Nähens ebenfalls umgesetzt wird, ist aus der DE 41 39 628 C1 bekannt. Bei diesen Nähinstrumenten ist eine Nadel mit zwei Spitzen erforderlich.

Ein weiteres chirurgisches Nähinstrument ist aus der US 4,935,027 (Yoon) bekannt. Dieses Nähinstrument ist sowohl für die konventionelle als auch für die minimalinvasive Chirurgie (MIC) geeignet. Es besteht aus einem äußeren und einem inneren Rohr, die gegeneinander axial verschiebbar sind, aus deren distalen (dem Operateur abgewandten, in den Körper eines Patienten einzuführenden) Enden zwei zangen- oder pinzettenartige Backen oder Haken hervorragen, die hohl sind und in deren Hohlraum das Nähgut geführt wird. Aus der Druckschrift geht hervor, daß beide Backen oder Haken beweglich sein können oder die eine Backe feststeht und nur die andere beweglich ist. Am proximalen (dem Operateur zugewandten) Ende befindet sich ein Griffstück, das teilweise mit dem äußeren Rohr und teilweise mit dem inneren Rohr fest verbunden ist. Über das Griffstück werden die Backen oder Haken bewegt. Innerhalb des inneren Rohrs befindet sich die Zuführung für das Nähgut. Als Nähgut sind die üblichen Fäden einsetzbar.

Mit diesem Nähinstrument können lediglich Einzelknopfnähte angelegt werden, wobei jede Naht mit einem auf das Gewebe hin verschiebbaren Knoten gesichert werden muß. Die Anlage der Knoten erscheint kompliziert und zeitaufwendig.

Aus der DE 31 41 647 A1 ist ein chirurgisches Instrument zum Anlegen von Klammern bekannt. Die Klammern werden mit Hilfe des Instruments aus Draht geformt. Das Instrument enthält einen langgestreckten Körper mit einem Handgriff am proximalen Ende. Am distalen Ende des Körpers ist eine feststehende Stützbacke mit einer Matrize angeordnet. In der Matrize ist eine Aushöhlung zum Klammerschluß vorgesehen. Weiterhin ist am distalen Ende des Körpers eine bewegliche Andrückbacke mit einem Klammeraufschieber angeordnet. Die Andrückbacke ist am Körper in Richtung der genannten Matrize hin- und hergehend bewegbar befestigt. Ferner weist das Instrument einen Antrieb zum Verstellen der Andrückbacke und des Klammeraufschiebers auf. Die bewegliche Andrückbacke enthält einen Kanal für die Zuführung des Drahts. An der Andrückbacke ist ein Messer in zum Austrittsende des Kanals rechtwinkliger Richtung hin- und hergehend bewegbar angeordnet. Das Messer dient zum Abtrennen von Drahtstücken und ist mit dem obengenannten Antrieb kinematisch verbunden.

Das bekannte Instrument ist für die minimalinvasive Chirurgie wegen seiner Konstruktion und Größe nicht geeignet.

Aufgabe der Erfindung ist, ein weiteres chirurgisches Nähinstrument der eingangs genannten Art vorzuschlagen, das insbesondere für die minimalinvasive Chirurgie geeignet sein soll. Das Nähinstrument soll daher durch einen Trokar bedienbar sein. Außerdem soll ein Faden vorgeschlagen werden, der mit diesem Nähinstrument vernähbar ist. Da die Anlage von Knoten zur Fixierung von Geweberändern in der minimalinvasiven Chirurgie erhebliche Probleme bereitet, soll der Faden in der Weise gestaltet sein, daß er nicht verknotet werden muß. Schließlich besteht eine weitere Aufgabe der Erfindung darin, für den vorgeschlagenen Faden eine Hantierungshilfe in Form eines Übergabehalters anzugeben.

Die Aufgabe wird erfindungsgemäß durch einen Faden mit den im kennzeichnenden Teil des ersten Patentanspruchs beschriebenen Merkmalen, durch die im vierten Patentanspruch beschriebene Kombination aus einem Übergabehalter und dem Faden und durch die im fünften Patentanspruch beschriebene Kombination aus einem chirurgischen Nähinstrument und dem Faden gelöst. In den Unteransprüchen sind bevorzugte Ausgestaltungen des Fadens und der Kombination gemäß Anspruch 5 angegeben.

Das erfindungsgemäße Nähinstrument stellt einen Applikator für den Faden dar; es weist einen Applikatorschaft auf, der aus einem Schaftinnenteil und einem gegenüber dem Schaftinnenteil axial verschiebbaren äußeren Schaftrohr besteht und an seinem proximalen Ende ein mehrteiliges Griffstück trägt. Der Applikatorkopf am distalen Ende des Applikatorschafts trägt zwei Applikatorbacken, die federelastisch sind und ohne Einwirkung einer Kraft eine V-förmige Stellung annehmen. Durch distales Verschieben des äußeren Schaftrohres werden die federelastischen Backen gegeneinander gedrückt, so daß sie eine im wesentlichen parallele Stellung einnehmen. Eine dieser Backen trägt an ihrem distalen Ende eine spitze Nadel, deren Länge maximal dem Innendurchmesser des äußeren Schaftrohres entspricht. In einer bevorzugten Ausführungsform ist die nicht mit der Nadel versehene Backe in proximaler Richtung zurückziehbar, so daß die Nadel nicht durch diese Backe abgedeckt wird. Dieselbe Wirkung wird erzielt, wenn die mit der Nadel bestückte Backe in distaler Richtung verschiebbar ist. Die erste dieser beiden Ausführungsformen hat den zusätzlichen Vorteil, daß die Stellung der Nadel im Operationssitus konstant bleibt und keine regulierende Bewegung des Nähinstruments durch den Chirurgen erforderlich ist.

Der erfindungsgemäße Faden besteht aus einem Fadenabschnitt, der an seinen beiden Enden ein Verschlußunterteil und ein Verschlußoberteil trägt. Die beiden Verschlußteile greifen ineinander und lassen sich in dieser Stellung verriegeln. Das Verschlußunterteil ist so geformt, daß es sich auf die Nadel des Nähinstruments aufsetzen läßt. Die zu vernähenden Geweberänder werden von der Nadel durchstochen und auf das Verschlußunterteil gedrückt; in dieser Stellung läßt sich das Verschlußoberteil aufsetzen und verriegeln, so daß die Geweberänder fixiert sind.

Mit dem erfindungsgemäßen Übergabehalter wird das Aufsetzen des Faden-Verschlußuntertells auf die Nadel erleichtet. Die für eine Operation benötigte Anzahl von Fäden kann zuvor in eine entsprechende Anzahl von Übergabehaltern eingesetzt werden, so daß kein Zeitverzug durch das Aufsetzen des Verschlußunterteils auf die Nadel entsteht.

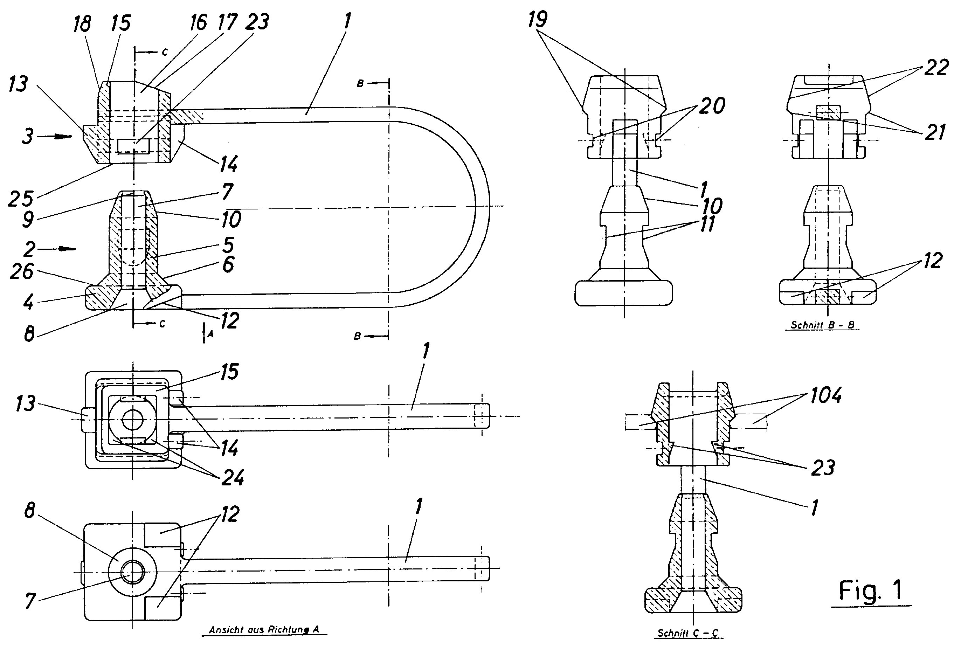

Besonders bevorzugte Ausführungsformen des Nähinstruments, des Übergabehalters und des Fadens werden nachfolgend anhand von Figuren näher beschrieben. Es zeigen:
**Fig. 1** den Faden mit seinen Verschlußteilen,
**Fig. 2** den Übergabehalter für den Faden,
**Fig. 3** den Faden im Übergabehalter beim Beladen des Nähinstruments,
**Fig. 4** eine Gesamtansicht des Nähinstruments,
**Fig. 5** den Griff des Nähinstruments mit Schnitten durch die Programmkulissenbahnen,
**Fig. 6** den Kopf des Nähinstruments mit eingelegtem Faden in Einsatzstellung,
**Fig. 7** den Kopf des Nähinstruments mit eingelegtem Faden,
**Fig. 8** den Kopf des Nähinstruments mit eingelegtem Faden nach dem Laden oder beim Einbringen in den Operationssitus durch den Trokar bzw. nach dem Durchstechen und Aufladen von Geweberändern,
**Fig. 9** den Kopf des Nähinstruments mit eingelegtem Faden und verriegelten Verschlußteilen,
**Fig. 10** den Kopf des Nähinstruments bei Freigabe der geschlossenen Naht,
Der Faden
**Fig. 1** zeigt den Faden, der aus resorbierbarem oder nicht resorbierbarem Kunststoff bestehen kann. Der Faden umfaßt den Fadenabschnitt 1, dessen Querschnittsform und Querschnittsfläche sowie dessen Länge entsprechend den anatomischen Voraussetzungen frei gewählt werden können. Am ersten Ende des Fadenabschnitts 1 befindet sich das Verschlußunterteil 2 und am zweiten Ende das Verschlußoberteil 3.

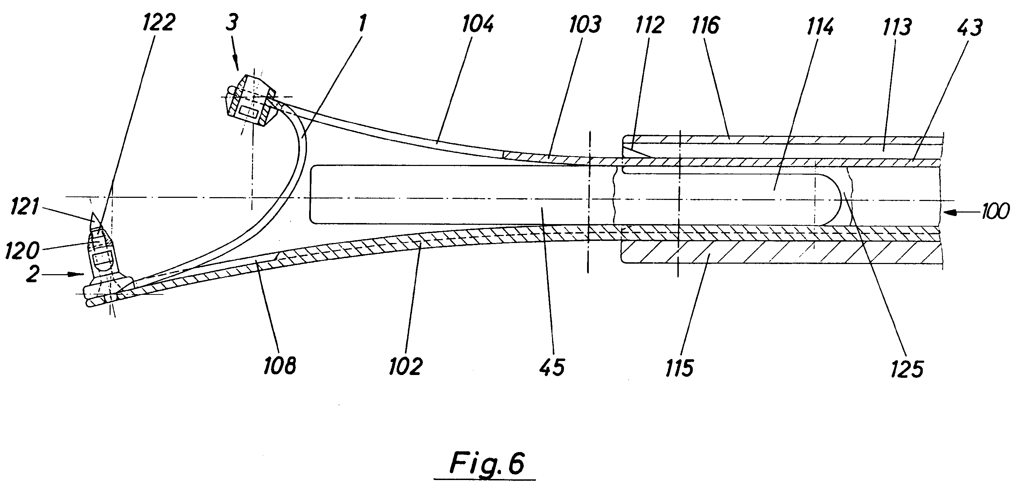

Das Verschlußunterteil 2 besteht aus einer mit einer zentralen kegeligen Bohrung 8 versehenen Platte 4, die auf ihrer Oberseite 26 unter Zwischenschaltung eines Übergangkegels 6 einen Hohlzylinder 5 trägt. Die Bohrung 7 im Hohlzylinder 5 fluchtet mit der Bohrung 8 in der Platte 4. Der freie Rand 10 des Hohlzylinders 5 ist im gleichen Winkel abgeschrägt wie die Spitze 121 der Nadel 120 auf der unteren Applikatorbacke 102, (vgl. **Fig. 6 - 10**) um ein problemloses Übergleiten und Aufladen von Gewebe zu ermöglichen. Der obere freie Rand 10 des Hohlzylinders 5 ist zur Bildung eines Wulstes 9 nach innen in die Bohrung 7 eingezogen. Dieser Wulst 9 hat die Aufgaben, durch sein Einrasten in den Einstich (122) unterhalb der Spitze (121) der Nadel (120) auf der unteren Applikator-Backe (102) das Verschlußunterteil (2) des Fadens (1) gegen unbeabsichtigtes Abrutschen von der Nadel (120) zu sichern.

In der Mantelfläche des Hohlzylinders 5 verlaufen parallel zur Platte 4 gegenüberliegend zwei Aussparungen 11, in die zur Verriegelung des Verschlusses die Nasen 23 in der Bohrung 16 des Verschlußoberteiles 3 einrasten.

Die Abschrägungen 12 an der Unterseite der Platte 4 gleiten synchron mit der Verschlußverriegelung auf die entsprechenden Abschrägungen 111 der Rippen 118 im Profilzylinder 101 des Applikatorkopfes 100 und entriegeln durch Abheben der Platte 4 von der unteren Applikatorbacke 102 die Fesselung des Wulstes 9 im Einstich 122 der Nadel 120, so daß nach Öffnen des Applikatormauls das Verschlußunterteil 2 leicht von der Nadel abgleiten kann.

Das Verschlußoberteil 3 besteht im wesentlichen aus einem Vierkantrohr 15 mit der Bohrung 16. Im unteren Teil der äußeren Mantelfläche trägt das Vierkantrohr 15 zu seiner Abstützung auf der Unterseite der oberen Applikatorbacke 103 drei nach unten abgeschrägte Rippen 13 und 14. Die Einzelrippe 13 ist auf der distalen Stirnseite des Vierkantrohres 15 angebracht und etwas breiter als die Rippen 14, um eine Auflage auf beiden Armen 104 der längsgeschlitzten oberen Applikatorbacke 103 zu sichern. Die beiden Rippen 14 liegen zu beiden Seiten des Fadens 1 an der proximalen Außenseite des Vierkantrohres 15 und stützen sich auf dem jeweils entsprechenden Arm 104 der oberen Applikatorbacke 103 ab.

Die beiden Seitenflächen der Bohrung 16 des Vierkantrohres 15 tragen die keilförmigen Nasen 23, die bei der Verschlußverriegelung in die Aussparungen 11 im Mantel des Hohlzylinders 5 des Verschlußunterteiles 2 einrasten.

Um die beim Übergleiten der Nasen 23 über den Mantel des Hohlzylinders 5 auftretenden Verformungswege im Verschlußoberteil 3 bereitstellen zu können, wurde als seine Grundstruktur ein Rohr 15 mit Vierkantquerschnitt gewählt, das sich unter innen angreifender temporärer Belastung in Richtung auf eine Kreisform aufdehnend verändern kann.

Nur so ist eine hinreichend große elastische Deformation des Verschlußoberteiles 3 erzielbar. Ein weiterer Vorteil dieser Konstruktion ist die Bildung von vier Kanälen 24 zwischen dem Hohlzylinder 5 des Verschlußunterteiles 2 und der Wand der Bohrung 16 im Verschlußoberteil 3, in die zur gewünschten Auflösung des resorbierbaren Kunststoffes Gewebeflüssigkeit eindringen kann.

Die beiden an den äußeren Seitenflächen des Vierkantrohres 15 waagerecht verlaufenden Rippen 19 dienen der lösbaren Fixierung des Verschlußoberteiles 3 in der oberen Applikatorbacke 103. Beim Beladen des Applikators mit dem Faden spurt sein Verschlußoberteil 3 in den Durchbruch 105 der oberen längsgeschlitzten Applikatorbacke 103 ein und drückt die beiden Arme 104 durch die Keilwirkung der oberen Abschrägungen 22 bei Anlage an den Schrägen 106b nach lateral auseinander, bis sie nach Durchtritt der Rippen 19 wieder federnd zusammenfallen, so daß das Verschlußoberteil 3 durch die Rippen 19, 13 und 14 in der oberen Applikatorbacke 103 gefangen ist.

Die Abschrägung 17 des Verschlußoberteiles 3 läuft bei der Verschlußverriegelung gegen eine entsprechende Abschrägung 112 im Profilzylinder 101 des Applikatorkopfes 100. Durch das distale Vorschieben des Profilzylinders 101 wird das Verschlußoberteil 3 aus dem in der **Fig. 1**, Schnitt C-C, gestrichelt dargestelltem Profil des Durchbruches 105 der oberen Backe 103 hinausgedrückt. Dieser Vorgang wird erleichtert durch die untere Abschrägung 21 der Rippen 19 des Verschlußoberteiles 3, die auf der entsprechenden oberen Schräge 106a im Durchbruch 105 unter lateralem Aufspreizen der Arme 104 abgleiten. Durch das Öffnen des Applikatormaules wird das Verschlußoberteil 3 - verriegelt mit dem Unterteil 2 - ohne weiteren Kraftaufwand aus der oberen Applikatorbacke 103 freigegeben.

Da das ebenfalls nicht mehr fixierte Verschlußunterteil 2 leicht von der Nadel 120 abgleitet, ist das Legen der Einzelknopfnaht abgeschlossen.

In die nun offenen Bohrungen 7 und 8 des Verschlußunterteiles 2 kann Sekret des Lagergewebes eindringen und von hier aus zur Auflösung des Verschlusses beitragen, sofern der Faden aus einem resorbierbaren Material besteht.

Die Abschrägung 18 der oberen distalen Stirnseite des Vierkantrohres 15 dient dem leichteren Einspuren des Verschlußoberteiles 3 in den Durchbruch 105 in der oberen Applikatorbacke 103.

Die unterhalb der Rippen 19 parallel zu ihnen verlaufenden Nuten 20 dienen der formschlüssigen (in Querrichtung) und reibschlüssigen (in Längsrichtung) Fixierung des Verschlußoberteiles 3 in der U-förmigen Aussparung 32 im Übergabehalter 30.

### Übergabehalter

Es wäre sehr zeitaufwendig, Konzentration und Geschicklichkeit erfordernd sowie fehlerträchtig, wenn während einer Operation die winzigen Fäden 1 mit ihren Verschlußteilen 2 und 3 per Hand oder Pinzette auf die Nadel 120 der unteren Applikatorbacke 102 bzw. in den Durchbruch 105 der oberen Applikatorbacke 103 gefädelt werden müßten.

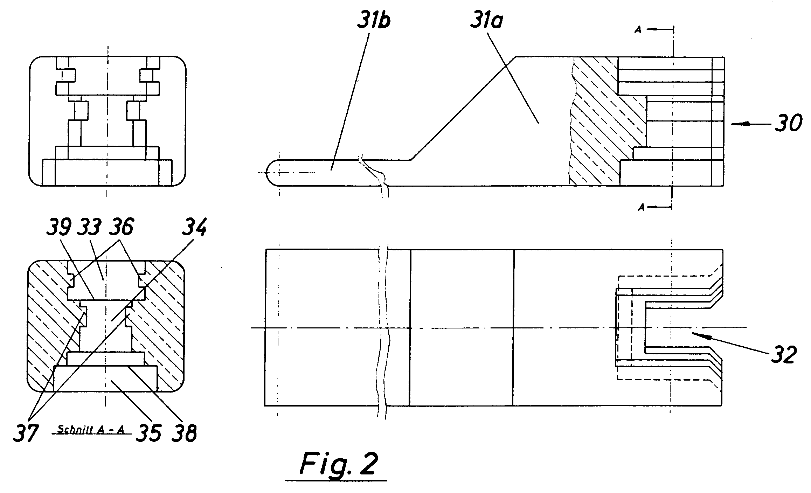

Deshalb wird als Handhabungshilfe ein in **Fig. 2** dargestellter, etwa 15 mm langer Übergabehalter 30 für die Beladung des Applikators mit dem Faden vorgeschlagen. Mehrere Übergabehalter 30 können mit den von ihnen gehaltenen Fäden 1 und den Verschlußteilen 2 und 3 nach **Fig. 1** in Magazinen oder Streifenkassetten steril verpackt passend zu ihren jeweiligen Applikatoren mit 5 mm, 7 mm und 10 mm Durchmesser im Operationssaal bevorratet werden.

Der einstückige Übergabehalter 30 besteht gemäß **Fig. 2** aus einem in seiner Höhe dem Fadenverschluß angepaßten gabelförmigen Kopf 31a mit der die Verschlußteile 2 und 3 aufnehmenden Aussparung 32.

Der mit dem gabelförmigen Kopf 31a verbundene Griff 31b ist wesentlich dünner und relativ biegeweich ausgeführt, um während des Einbaues des Fadens 1 in dessen Applikator einer deformierenden Kraftübertragung (Biegemomente) vom Übergabehalter 30 auf die Nadel 120 und auf die untere Applikatorbacke 102 zu begegnen.

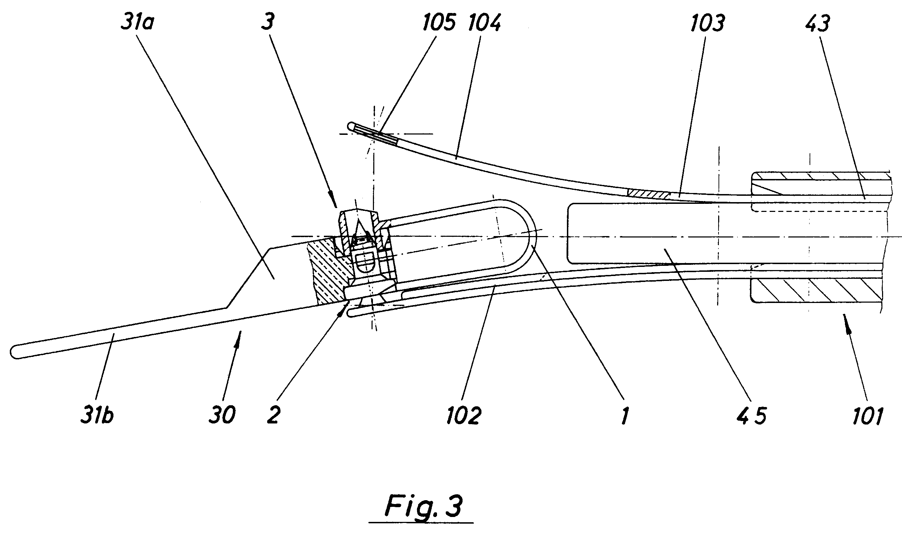

Bei der Konfektionierung von Übergabehalter 30 und Faden werden die Verschlußteile 2 und 3 des Fadens 1 von der Stirnseite des gabelförmigen Kopfes 31a aus reibschlüssig in die Aussparung 32 so eingedrückt, daß der Faden - wie in **Fig. 3** dargestellt - frei vor der Stirnseite des gabelförmigen Kopfes liegt.

Dabei wird das Verschlußoberteil 3 durch die Rippen 36 der oberen Aussparung 33 in den Nuten 20 gehalten. Die Verbindung ist quer zu den Nuten 20 formschlüssig. Die reibschlüssige Fixierung des Verschlußoberteiles 3 in Richtung der Nuten 20 ist dagegen über alle seine Berührungsflächen mit der oberen Aussparung 33 im gabelförmigen Kopf 31a realisiert.

Das Verschlußunterteil 2 des Fadens 1 wird durch die Rippen 37 in der mittleren Aussparung 34 des gabelförmigen Kopfes 31a festgehalten. Die dabei in den Aussparungen 11 des Hohlzylinders 5 liegenden Rippen 37 blockieren das Verschlußunterteil 2 quer zu ihrer Längsrichtung formschlüssig. Die reibschlüssige Sicherung in Richtung der Gabelöffnung erfolgt wieder über alle Berührungsflächen des Verschlußunterteiles 2 mit den Bereichen 34 und 35 der Aussparung 32.

Nach der Beladung des Übergabehalters 30 liegt die untere Stirnfläche 25 des Verschlußoberteiles 3 auf der Fläche 39 der oberen Aussparung 33 und die obere Fläche 26 der Platte 4 auf der Fläche 38 der unteren Aussparung 35 auf. In dieser Lage relativ zueinander sind die Verschlußteile 2 und 3 des Fadens 1 zwar - wie die **Fig. 3** zeigt - schon etwas eingespurt, aber nicht verriegelt. Auch axiale Kräfte, wie sie bei der Übernahme der Verschlußteile 2 und 3 in die Applikatorbacken 102 und 103 auftreten, können aufgrund dieser Abstützung die relaLage der Verschlußteile 2 und 3 zueinander nicht verändern.

### Nähinstrument bzw. Applikator für den Faden

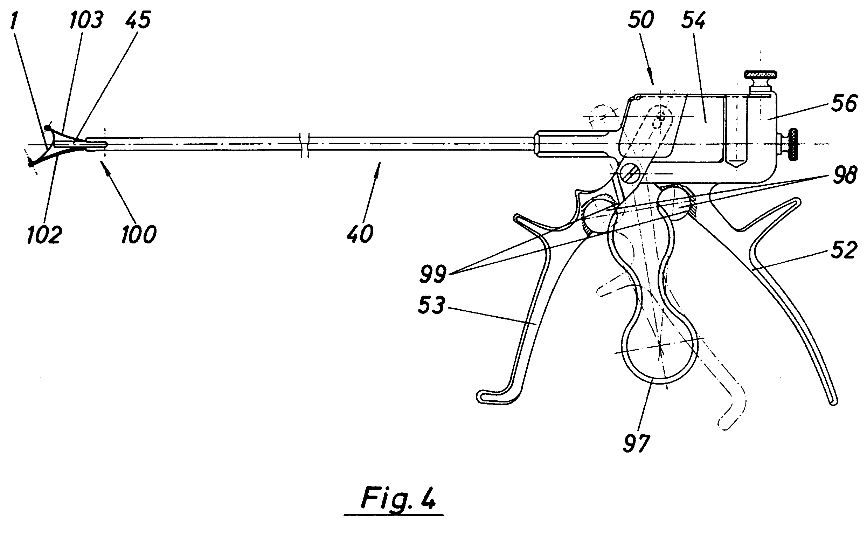

Der Applikator nach **Fig. 4** zum Legen der vorstehend beschriebenen Einzelknopfnähte besteht im wesentlichen aus drei Baugruppen:
- Dem Schaft 40 mit dem Schaftrohr 41 und den Innenteilen 42 und 43,
- dem Griff 50 mit dem Griffkopf 51 und dem mit ihm einstückig verbundenen Handteller - Griffteil 52 sowie mit dem gelenkig angeschlossenen Finger - Griffteil 53 am proximalen Schaftende und
- dem Applikatorkopf 100 mit der oberen geschlitzten Backe 103, der unteren nadelbestückten Backe 102 und dem auf diesen Backen verschiebbaren Profilzylinder 101 am distalen Schaftende.

Im folgenden wird der mechanische Aufbau dieser drei Instrumentenbaugruppen näher beschrieben.

### Der Applikatorschaft

Wie in den **Fig. 4, 5** und **8** gezeigt, besteht der Applikatorschaft 40 aus dem Rohr 41, das - wie später beschrieben - gegen Verdrehen gesichert durch Betätigen des Finger-Griffteiles 53 auf dem Schaftinnenteil 42 und der geraden Verlängerung 43 der oberen Backe 103 gleitend translatorisch nach distal und promixal bewegt werden kann.

Die gerade Verlängerung 43 der oberen Backe 103 gleitet ihrerseits in einer nach oben offenen Längsnut 44 des Schaftinnenteiles 42, wenn die obere Backe 103 von oder zu ihrer Korrespondenzstellung mit der Nadel 120 auf der unteren Backe 102 relativ zu dieser bewegt wird. Dabei stützen sich die oberen lateralen Kanten der geraden Verlängerung 43 der oberen Backe 103 an der Innenfläche des Schaftrohres 41 ab.

An seinem distalen Ende ist das Schaftinnenteil 42 zu einem Stempel 45 mit Rechteckprofil modifiziert. Dieser Stempel 45, dessen Abmessungen im Zusammenhang mit der Darstellung des Applikatorkopfes 100 näher beschrieben werden, dient bei der Instrumentenmontage zur leichteren Einführung der nach außen aufspringenden Backen 102 und 103 in das Schaftrohr 41 und in die Führungsnuten der Backen im Applikatorkopf.

Bevor es am distalen Ende in den Stempel 45 übergeht, erhält das Schaftinnenteil 42 - der Nut 44 diametral gegenüberliegend - eine Abplattung 46 mit einer Längsnut 47. Hier wird - z.B. mittels Laserschweißung - die untere Backe 102 am Schaftinnenteil 42 befestigt.

### Applikatorgriff

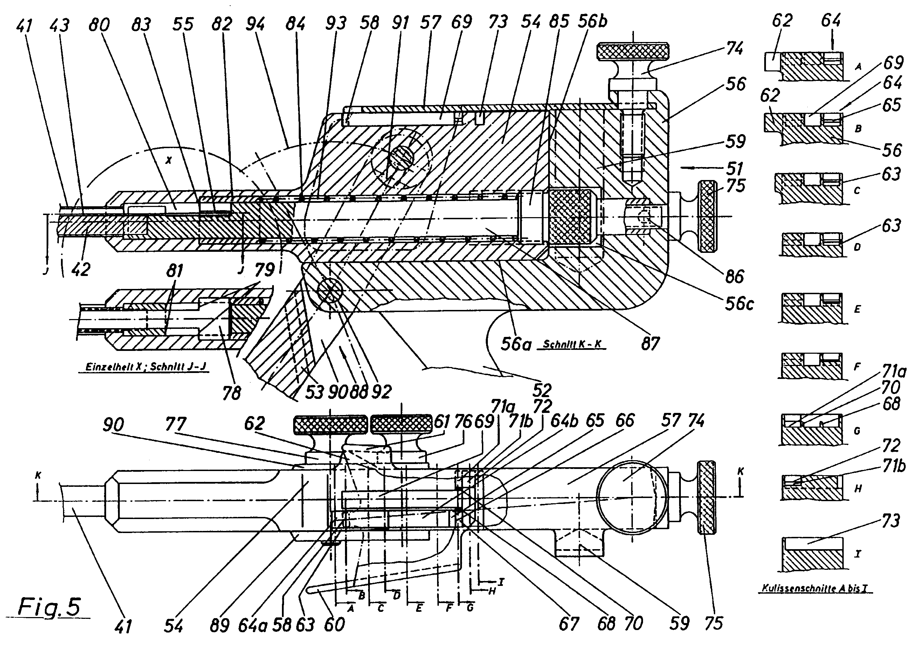

Der Applikatorgriff 50 ist in **Fig. 5** dargestellt; er dient zum sicheren Halten und Führen des Instrumentes in den und im Operationssitus und durch Betätigung des Finger-Griffteiles 53 und des Federhebels 57 zur Aktivierung der zum Nahtlegen erforderlichen Funktionen des distalen Applikatorkopfes 100.

Der Kreuzkopf 54, der fest mit dem Schaftrohr 41 verbunden ist und dieses gegen Verdrehen sichert, wird durch proximales Ziehen am Finger-Griffteil 53 gegen die Kräfte der Federn 84 und 97, geführt zwischen den Wangen 89 und 90 der Gabel 88 am Finger-Griffteil 53, auf der Gleitfläche 56a des Winkelstückes 56 und auf der Verlängerung 87 des Schaftinnenteiles 42, distalwärts verschoben. Bei Reduzierung oder Aufhebung der Krafteinwirkung auf das Finger-Griffteil 53 wird der Kreuzkopf 54 durch die in den Federn 84 und 97 gespeicherte Energie wieder nach proximal zurückbewegt.

Das Handteller-Griffteil 52 ist einstückig mit dem Winkelstück 56 des Griffkopfes 51 verbunden.

Das Finger-Griffteil 53 ist durch die Rändelschraube 77 in den Wangen 89 und 90 und der Bohrung 92 schwenkbar an das Winkelstück 56 des Griffkopfes 51 angelenkt.

Die Verbindung der Wangen 89 und 90 des Fingergriffteiles 53 mit dem Kreuzkopf 54 erfolgt mit der Rändelschraube 76. Sie ist (von Hand lösbar) in den Kreuzkopf 54 eingeschraubt und ihre zylindrischen Schaftteile gleiten während der Schwenkung des Finger-Griffteiles 53 um den Winkel 94 in den Langlöchern 91 der Wangen 89 und 90.

Das Schaftinnenteil 42, das am distalen Ende die untere nadelbestückte Applikatorbacke 102 trägt, ist über die Verlängerung 87 mit dem Winkelstück 56 und so auch mit dem Handteller-Griffteil 52 durch die Rändelschraube 75 fest, aber lösbar, verbunden. Daher sind keine korrigierenden Hand- oder Armbewegungen erforderlich, wenn nach Positionierung der Nadel 120 unter einen Inzisionsrand die weiteren Funktionen des Applikatorkopfes 100 aktiviert werden, da Hand und Nadel relativ zueinander ortsfest bleiben.

Um die Lage der Applikatorbacken 102 und 103 relativ zu ihren Führungsnuten 109 und 110 im Profilzylinder 101 des Applikatorkopfes 100 exakt zu sichern, besitzt die proximale Stirnseite der Verlängerung 87 eine radiale Indexnut, in die der im Winkelstück 56 fixierte Stift 86 eingreift.

In der Bohrung 93 des Kreuzkopfes 54 liegt gleitend verschiebbar, an der Fläche 83 des Absatzes anschlagend, eine quergeschlitzte Hülse 82. Sie ist belastet durch die vorgespannte Druckfeder 84, deren proximales Ende sich auf der von Hand lösbaren, gerändelten Hohlschraube 85 abstützt, die in das Innengewinde am proximalen Ende der Bohrung 93 eingeschraubt ist. Die Verlängerung 87 des Schaftinnenteiles 42 in der Achse der Bohrung 93 wird von der quergeschlitzten Hülse 82, der Druckfeder 84 und der Hohlschraube 85 gleitend umschlossen.

In der Nähe ihres distalen Endes hat die Verlängerung 87 die Aussparung 80, deren Grundfläche unter dem Querhaupt 78 am proximalen Ende der geraden Verlängerung 43 der oberen Backe 103 liegt. Die gerade Verlängerung 43 der oberen Backe 103 ist - wie bereits beschrieben - in einer Längsnut 44 des Schaftinnenteiles 42 zwischen dem Profilzylinder 101 des Applikatorkopfes 100 und der Aussparung 80 in der Verlängerung 87 des Schaftinnenteiles 42 in Längsrichtung verschiebbar geführt. Das Querhaupt 78, die obere Backe 103 und ihre gerade Verlängerung 43 haben gleiche Dicke und sind einstückig aus federhartem nichtrostendem Stahlblech hergestellt.

In der in **Fig. 6** dargestellten Ausgangsposition des Arbeitszyklus des Applikators liegt das Querhaupt 78, wie in der **Fig. 5** eingezeichnet, am proximalen Ende der Aussparung 80 in der Verlängerung 87. Die beiden Arme des Querhauptes 78 werden oben, unten und an den proximalen Flächen von den beiden Querschlitzen 55 in der Hülse 82 im geringem Spiel umschlossen. Die distalen Flächen 79 des Querhauptes 78 liegen dann wie die quergeschlitzte Hülse 82 vor der Fläche 83 des Absatzes in der Bohrung 93 des Kreuzkopfes 54.

Wird nun der Kreuzkopf 54 durch Anziehen des Finger-Griffteiles 53 relativ zum Schaftinnenteil 42 und zu dessen Verlängerung 87 nach distal verschoben, dann folgt zunächst auch das Querhaupt 78 in der Aussparung 80 der Verlängerung 87 diesem Vorschub, da die Vorspannung der Druckfeder 84 so bemessen ist, daß die Reibung der oberen Backe 103 im Profilzylinder 101 des Applikatorkopfes 100 sowie die Reibung ihrer geraden Verlängerung 43 in der Längsnut 44 des Schaftinnenteiles 42 überwunden wird. Dadurch gleitet der Profilzylinder 101 des Applikatorkopfes 100 mit der relativ zu ihm noch nicht bewegten oberen Backe 103 distalwärts über die untere Backe 102.

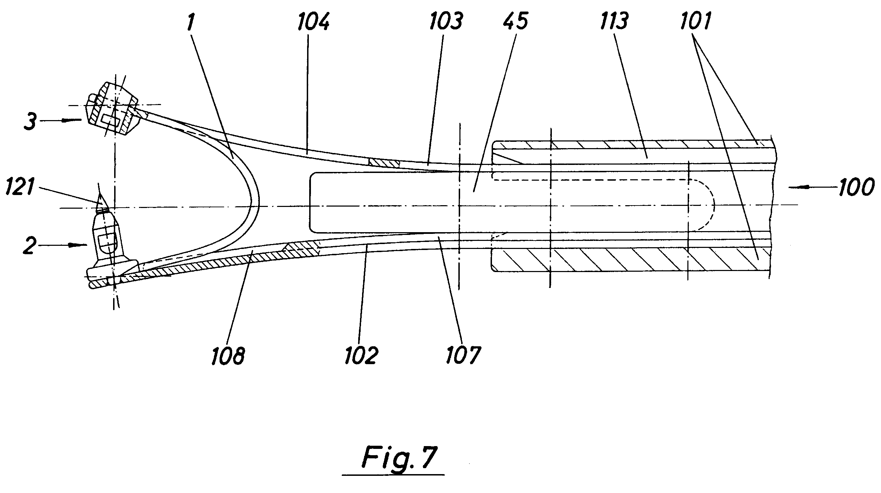

Dieser Funktionsabschnitt ist beendet, wenn die distale Anlagefläche 79 des Querhauptes 78 den distalen Anschlag 81 der Aussparung 80 in der Verlängerung 80 erreicht hat. Dann befindet sich die obere Backe 103 in der in **Fig. 7** gezeichneten Korrespondenz-Stellung relativ zu unteren Backe 102 und zur Nadel 120.

Bei weiterem distalen Vorschub des Kreuzkopfes 54 bleiben das Querhaupt 78 durch die Anlage an der distalen Anschlagfläche 81 und damit auch die quergeschlitzte Hülse 82 relativ zur Verlängerung 87 des Schaftinnenteiles 42 blockiert und die Druckfeder 84 wird unter Arbeitsaufnahme zusammengedrückt. Durch die Anlage des Querhauptes 78 an den Anschlagflächen 81 bleibt dabei die relative Lage der oberen Backe 103 zur unteren Backe 102 in Längsrichtung unverändert.

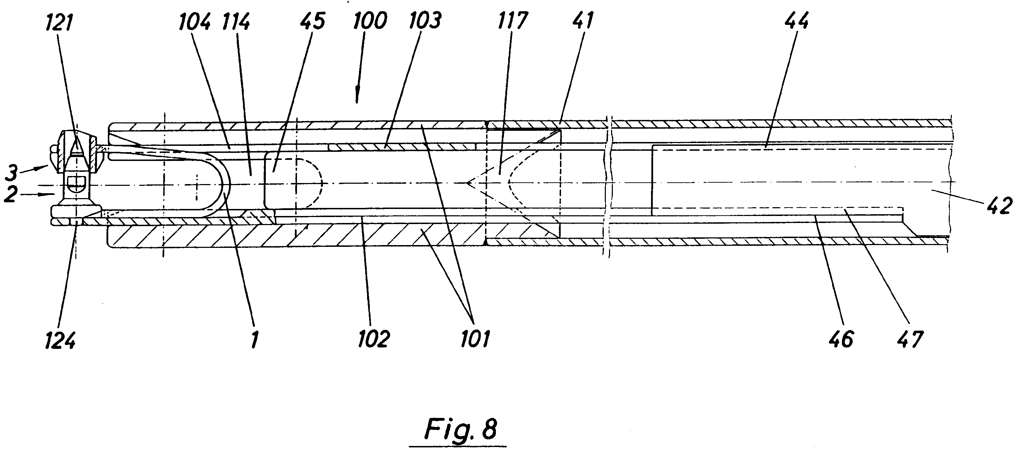

Nur die distalen Enden der Backen 102 und 103 bewegen sich aufeinander zu, bis die Endstellung des Arbeitszyklus nach **Fig. 8** erreicht ist: Durchstechen und Aufladen eines Geweberandes ist beendet.

Die in **Fig. 8** gezeigte Stellung des Applikatorkopfes 100 gilt auch für das Ende des Beladungsvorganges des Applikators mit dem Faden, wenn seine Verschlußteile 2 und 3 gesichert von den Backen 102 und 103 übernommen sind.

Wird der Fingerzug auf das Finger-Griffteil 53 zur Öffnung der Backen 102 und 103 reduziert, so wandern - angetrieben durch die gespannten Federn 84 und 97 - zunächst der Profilzylinder 101, das Schaftrohr 41 und der Kreuzkopf 54 nach proximal zurück, ohne daß sich in Längsrichtung an der relativen Lage von oberer Backe 103 zur unteren Backe 102 etwas ändert, da das Querhaupt 78 mit seinen Anlageflächen 83 noch unter der Last der gespannten Druckfeder 84 an den Flächen 81 in der Aussparung 80 anliegt. Die Bohrung 93 im Kreuzkopf 54 gleitet nach proximal über die geschlitzte Hülse 82 zurück, bis ihre distale Stirnfläche von der Anlagefläche 83 in der Bohrung 93 erreicht wird. Damit ist wieder die in **Fig. 7** dargestellte Backenposition im Applikatorkopf 100 hergestellt.

Wandert der Kreuzkopf 54 jetzt unter alleiniger Wirkung der Rückstellfeder 97 zwischen den Griffteilen 52 und 53 weiter nach proximal, so werden auch sowohl die Hülse 82 als auch das Querhaupt 78 von der Anlagefläche 83 in der Bohrung 93 nach proximal bewegt, bis der Kreuzkopf 54 - wie in der **Fig. 5** dargestellt - die senkrechte Anschlagfläche 56b im Winkelstück 56 erreicht. Die gerändelte Hohlschraube 85 liegt dann in der Bohrung 56c des Winkelstückes 56.

Zur Sicherung des Applikators gegen unbeabsichtigte Einleitung von Arbeitsschritten oder -zyklen ist im Griffkopf 51 eine Programmechanik vorgesehen. Sie besteht, wie in **Fig. 5** dargestellt, im wesentlichen aus den Kulissenbahnen 63 bis 73 im Kreuzkopf 54 und dem Federhebel 57 mit dem in den Kulissenbahnen gleitenden Zapfen 58. Der Federhebel 57 ist durch die Rändelschraube 74 horizontal schwenkbar mit dem Winkelstück 56 verbunden.

Zur Beschreibung der Programmechanik wird von der in **Fig. 5** dargestellten Draufsicht ausgegangen.

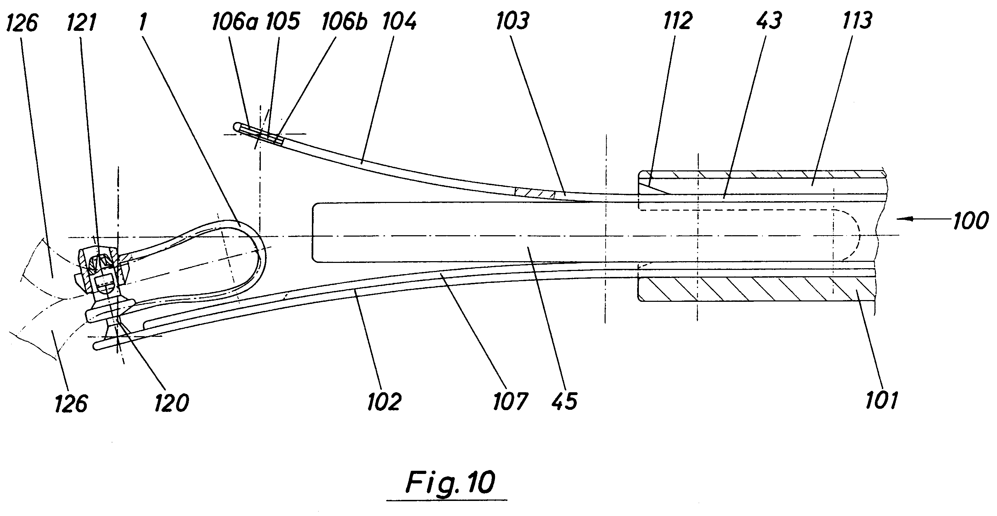

Der Zapfen 58 des Federhebels 57 befindet sich - strichpunktiert dargestellt - auf der unteren waagerechten Ebene 64a der Beladekulissenbahn 64. Die in **Fig. 10** dargestellten Positionen von Profilzylinder 101 und Backen 102 und 103 relativ zueinander entsprechen (ohne den dort eingezeichneten verriegelten Faden) dieser Ausgangsstellung der Programmkulisse.

Nach Aufhängen des Fadenverschlusses auf die Nadel 120 der unteren Backe 102 mittels des Übergabehalters 30 wird der Kreuzkopf 54 durch Anziehen des Finger-Griffteiles 53 nach distal bewegt. Der Profilzylinder 101 und die obere Backe 103 bewegen sich entsprechend in Richtung auf die Nadel 120 gleitend über die untere Backe 102 bis zu der in der **Fig. 3** dargestellten Anordnung. Dabei gleitet der Zapfen 58 des Federhebels 57 in der Kulissenbahn 64 in Richtung auf die Vertiefung 67. Der Zapfen 58 wird durch die nach unten gerichtete Federkraft des Federhebels 57 nach unten auf den Kulissengrund und durch die Wirkung der federnden Zunge 59 seitlich gegen die linke Kulissenwand gedrückt und so in dieser Kulissenbahn gefangen. Im Verlaufe des distalen Vorschubes des Kreuzkopfes 54 verläßt der Zapfen 58 die untere waagerechte Kulissenebene 64a und gleitet über die schräg ansteigende Bahn 64b und die Schwelle 65 bis auf die obere Ebene, die mit dem Auflaufen des Zapfens 58 auf die Kante 66 erreicht wird. Nach Überfahren der Kante 66 "fällt" der Zapfen 58 am Federhebel 57 in die Vertiefung 67 am proximalen Ende der Beladekulissenbahn. Die dieser Situation entsprechende Stellung des Applikatorkopfes 100 mit den von den Backen 102 und 103 gefaßten Verschlußteilen 2 und 3 des Fadens 1 zeigt die **Fig. 8**. Ein Zurückgleiten des Kreuzkopfes 54 ist jetzt nicht mehr möglich, auch wenn die Kraft auf das Finger-Griffteil 53 entfällt. Das Instrument kann - entsprechend **Fig. 8** beladen - in Erwartung des Einsatzes zur Seite gelegt oder direkt dem Operateur angereicht werden. Dieser führt den nach **Fig. 8** beladenen Applikatorkopf 100 durch eine transkutane Trokarhülse in den Operationsitus ein.

Um in den Arbeitszyklus zum Durchstechen und Aufladen der zu verbindenden Inzisionsränder zu gelangen, wird das Finger-Griffteil 53 so weit angezogen, daß die Anlage des Zapfens 58 an der Kante 66 aufgehoben ist und damit der Federhebel 57 durch Daumendruck auf die Druckplatte 60 leicht in Richtung der Arbeitskulissenbahn 69 geschwenkt werden kann. Dabei gleitet der angeschrägte Zapfen 58 des Federhebels 57 über die angefaste Rippe 68, "fällt" von dort zurück auf die waagerechte untere Ebene der Arbeitskulissenbahn 69 und ist damit in dieser Bahn gefangen. Durch Reduzierung der Zugkraft auf das Finger-Griffteil 53 läuft der Kreuzkopf 54, der Profilzylinder 101 und die obere Backe 103 nach proximal zurück, und die Ausgangsstellung der Applikatorbacken 102 und 103 mit dem eingelegten Faden ist, wie in der **Fig. 6** dargestellt, erreicht.

Die durch die Krümmung der unteren Backe 102 schräg nach distal weisende Nadel 120 erleichtert ebenso wie die zurückgesetzte obere Backe 103 das Unterfahren eines Geweberandes und das Ansetzen der Nadel 120 an der gewünschten Durchstichstelle. Eine gerade statt der gebogenen unteren Backe 102 erlaubt zwar die Benutzung kürzerer Fäden 1 und erzeugt geringere Reibungskräfte in den Rührungsnuten 110, verliert aber den Vorteil der leichteren Gewebeübernahme.

Nach Ansetzen der Nadel 120 unter die Durchstichstelle im Gewebe wird durch Anziehen des Finger-Griffteiles 53 - wie beschrieben unter Beibehaltung der Nadelposition ohne ausgleichende Arm- oder Handbewegungen - zunächst die obere Backe 103, wie in der **Fig. 7** gezeigt, vorgeschoben und anschließend unter Annäherung der Verschlußteile 2 und 3 in den Backen 102 und 103 durch weiteren distalen Vorschub des Profilzylinders 101 das Gewebe durchstochen und so auf das Verschlußunterteil 2 aufgeladen. Während dieser Arbeitszyklen zum Durchstechen und Aufladen des Gewebes gleitet der Zapfen 58 des Federhebels 57 zwischen den Anschlägen am distalen und proximalen Ende der Arbeitskulissenbahn 69 entsprechend der Bewegung des Kreuzkopfes 54 hin und her.

Sind beide Geweberänder auf das Verschlußunterteil 2 aufgeladen, muß der Fadenverschluß verriegelt werden. Das geschieht sinnvollerweise direkt im Anschluß an das Aufladen des zweiten Inzisionsrandes. Dazu wird mit dem Daumen - wie vorher beschrieben - der Federhebel 57 in die Richtung der Verriegelungskulissenbahn 71 geschwenkt. Der Zapfen 58 am Federhebel 57 gleitet dabei an der proximalen Stirnseite der Arbeitskulissenbahn 69 entlang, überfährt die geschrägte Rippe 70 und "fällt" anschließend in die Vertiefung 71a in der Verriegelungskulissenbahn 71. Damit ist zunächst das Instrument und der Applikatorkopf 100 in dem in der **Fig. 8** gezeigten Zustand gesichert.

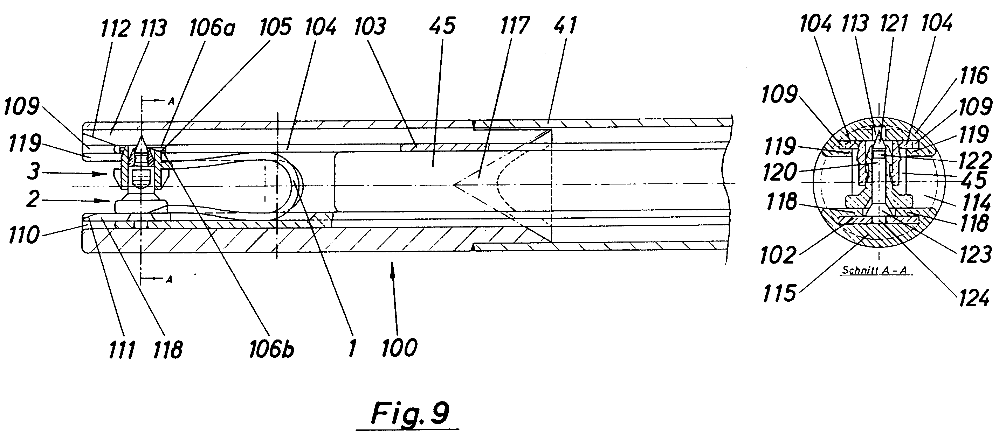

Durch weiteres Anziehen des Finger-Griffteiles 53 wird das Schaftrohr 41 und mit ihm der Profilzylinder 101 weiter nach distal bis in die in der **Fig. 9** dargestellte Position über die Backen 102 und 103 geschoben. Der Zapfen 58 am Federhebel 57 gleitet während dieses Vorgangs aus der Vertiefung 71a über die schräge Rampe 71b der Verriegelungskulissenbahn 71, um nach vollständiger Überfahrung der oberen Kante 72 in die Rücklaufkulissenbahn 73 zu "fallen".

Durch die Rückstellkraft der federnden Zunge 59 am Federhebel 57 springt dieser in eine Stellung zurück, die durch eine entsprechende Abschrägung seines proximalen Endes hinter der als Drehpunkt wirkenden Rändelschraube 74 festgelegt ist. In dieser Stellung des Federhebels 57 liegt sein Zapfen 58 seitlich neben dem Kreuzkopf 54.

Die Einzelvorgänge bei der Verriegelung des Fadenverschlusses, wie sie in **Fig. 9** gezeigt ist, werden im Zusammenhang mit der Beschreibung des Applikatorkopfes 100 im nächsten Abschnitt dargestellt. Hier wird nur festgehalten, daß synchron mit der Verschlußverriegelung des Fadens 1 auch die Klemmungen der Verschlußteile 2 und 3 in den Applikatorbacken 102 und 103 aufgehoben werden.

Wird das Finger-Griffteil 53 bei nachlassender Zugkraft nach vollzogener Verschlußverriegelung durch die Rückstellkraft der Federn 84 und 97 in Richtung Ausgangsstellung zurückbewegt, so gleitet der Kreuzkopf 54 nach proximal unter dem Federhebel 57 hindurch. Dabei liegen die Zapfen 58 seitlich neben der Daumenseite des Kreuzkopfes 54 und die Abkantung 61 auf der gegenüberliegenden Seite. Der Profilzylinder 101 läßt durch sein Zurückgleiten die Backen 102 und 103 V-förmig auseinanderspreizen und die fertige Einzelknopfnaht wird entsprechend **Fig. 10** freigegeben.

Gegen Ende dieses Rückstell- und Freigabevorganges läuft die Nase 62 an der daumenabgewandten Seite des Kreuzkopfes 54 mit ihrer flachen Abschrägung unter die distale senkrechte Stirnseite der Abkantung 61 des Federhebels 57 und zwingt damit den Federhebel gegen die Rückstellkraft der federnden Zunge 59 zu einer Schwenkung in die in der **Fig. 5** dargestellten Ausgangsposition. Durch die Abschrägung der seitlichen Führungsrippe 63 wird das Aufgleiten des ebenfalls angeschrägten Zapfens 58 und das Anheben des Federhebels 57 vor dem "Rückfall" in die Beladekulissenbahn 64 erleichtert.

Nach der Freigabe der Einzelknopfnaht mit den von ihr umschlungenen Inzisionsrändern 126 befindet sich der Applikatorkopf 100 in der in den **Fig. 6** und **10** gezeichneten Stellung - allerdings ohne den dort jeweils dargestellten Faden - im Operationssitus. Der Operateur könnte jetzt den Applikatorkopf 100 unter federndem Zusammengehen der Backen 102 und 103 einfach durch das Lumen der Trokarhülse extrahieren. Das könnte jedoch zur Beschädigung der ungeschützten Nadel 120 führen.

Deshalb ist in der Beladekulissenbahn die Schwelle 65 vorgesehen. Der Operateur zieht das Finger-Griffteil 53 so weit nach proximal bis er beim Anlaufen des Zapfens 58 an die Schwelle 65 einen Druckpunkt wahrnimmt. Die Backen 102 und 103 befinden sich dann etwa in der in der **Fig. 8** gezeigten Stellung (ohne Faden) vor dem Profilzylinder 101. Die Nadel 120 ist jetzt gut geschützt durch die Lage ihrer Spitze 121 in dem Durchbruch 105 der oberen Backe 103 und unmittelbar vor der distalen Stirnseite des Profilzylinders 101, befindet sich also bei der Extraktion durch die Trokarhülse im "Windschatten" des Applikatorkopfes 100. Nach dem Auszug des Applikatorkopfes aus der Trokarhülse kann das Finger-Griffteil 53 entlastet werden, so daß der Kreuzkopf 54 bis zum Anschlag an der Fläche 56b zurückläuft. Der Zapfen 58 des Federhebels 57 wandert dabei von der Schwelle 65 bis in die Ausgangsposition nach **Fig. 5** zurück, wobei er - wie auf dem Hinweg bis zur Schwelle 65 - in der Beladekulissenbahn 64 geführt bleibt.

Damit ist das Programm für das Legen einer Einzelknopfnaht vollständig durchlaufen und der Applikator steht zu erneuter extrakorporaler Beladung mit einem Faden zur Verfügung.

### Applikatorkopf

Der Applikatorkopf 100 besteht im wesentlichen aus der oberen federelastischen Backe 103, der unteren mit der Nadel 120 bestückten Backe 102, dem zwischen ihnen liegenden Stempel 45 und dem über diese Teile nach distal und proximal verschiebbaren Profilzylinder 101. Die relative Lage dieser Komponenten zueinander bei der Beladung des Instrumentes und während der Applikation einer Einzelknopfnaht ist in den **Fig. 3, 6, 7, 8, 9** und **10** dargestellt.

Das distale Ende des Schaftrohres 41 ist axial fluchtend verlängert durch den Profilzylinder 101, der den gleichen Außendurchmesser wie das Schaftrohr 41 hat.

Im distalen Ende des Profilzylinders 101 befindet sich symmetrisch zu dessen Längsachse der Querschlitz 114, so daß eine Gabel mit dem Oberteil 116 und dem Unterteil 115 entsteht. In diesen Querschlitz 114 gleiten die Geweberänder bei entsprechender Stellung der Backen 102 und 103 ein.

Fluchtend mit seiner Achse ist im Profilzylinder 101 ein Rechteckdurchbruch 125 vorhanden, in dem der Stempel 45 gleiten kann.

Der Rechteckdurchbruch 125 ist im oberen Teil durch die Führungsnuten 109 nach lateral erweitert, so daß sich im Gabeloberteil 116 über dem Querschlitz 114 die Längsrippen 119 ausbilden.

In der Längsachse der unteren Fläche des Rechteckdurchbruchs 125 liegt die T-förmige untere Nut 110, die unter dem Querschlitz 114 die Längsrippen 118 erzeugt.

In der Längsachse der oberen Begrenzungsfläche des Rechteckdurchbruches 125 ist in den Profilzylinder 101 eine weitere Längsnut 113 als Freistich für die Nadel 120 eingearbeitet.

Die obere Backe 103 ist, wie bereits geschildert, zusammen mit der geraden Verlängerung 43 und dem Querhaupt 78 einstückig aus federhartem, nichtrostendem Stahlblech gefertigt.

Nahe ihrem distalen Ende befindet sich in der oberen Backe 103 der viereckige Durchbruch 105 zur Aufnahme des Verschlußoberteiles 3. Damit dieses Oberteil 3 am Faden übernommen und federnd gehalten werden kann, ist die obere Backe 103 bis etwa zur Hälfte der frei aus dem Profilzylinder 101 hinausragenden Länge in der Längsachse senkrecht zur Oberfläche geschlitzt.

Proximal des Durchbruchs 105 ist der Schlitz zwischen den beiden Backenarmen 104 so breit, daß der Faden in ihm Platz findet. Die Weite des Schlitzes distal des Durchbruches 105 geht dabei aber gegen Null, da sich hier die beiden Backenarme 104 der oberen Backe 103 gegenseitig abstützen, um den Durchbruch 105 zum Einspuren des Verschlußoberteiles 3 genügend weit offen zu halten.

Die Längsseite des Durchbruches 105 in jedem der beiden Backenarme 104 hat je eine obere 106a und untere Schräge 106b, die das Ein- und Ausklinken der Rippen 19 am Verschlußoberteil 3 des Fadens erleichtern. Da das Ausklinken des Verschlußoberteiles 3 beim Verriegeln des Verschlusses ein seitliches Ausweichen der Backenarme 104 innerhalb der oberen Führungsnuten 109 erfordert, muß die obere Backe 103 am proximalen Ende des Schlitzes beginnend nach distal fortschreitend schmaler werden, bis - wie es im Schnitt A-A der **Fig. 9** dargestellt ist - genügend seitlicher Freiraum zur Backenaufweitung bzw. zur lateralen Auspreizung der Arme 104 zur Verfügung steht.

Die untere Backe 102 ist aus federhartem Edelstahl hergestellt und hat entsprechend ihrer unteren Führungsnut 110 einen T-förmigen Ausgangsquerschnitt. Dieser Querschnitt wurde gewählt, um das äquatoriale Widerstandsmoment der unteren Backe 102 in Vergleich zu oberen Backe 103 zu vergrößern. Dadurch kann die untere Backe 102 wesentlich höhere Biegemomente aufnehmen, die eventuell bei der Gewebemanipulation auftreten können.

Das proximale Ende der unteren Backe 102 ist mit dem distalen Ende des Schaftinnenteiles 42 durch Fixierung in dessen Längsnut 47 und auf dessen Abplattung 46 unlösbar - z.B. durch Laserpunktschweißung - verbunden.

Der T-förmige Ausgangsquerschnitt der unteren Backe 102 ist an ihrem distalen Ende zur Anbringung der Nadel 120 und zur Unterbringung eines Teiles des Fadens 1 modifiziert. Am äußeren distalen Ende der unteren Backe 102 ist die Längsrippe 107 vollständig entfernt, um eine größere ebene Auflage für die Nadel 120 zu erhalten und darüberhinaus diese Nadel noch etwas verlängern zu können. Daran anschließend erstreckt sich nach proximal die Längsnut 108 in der Rippe 107 der unteren Backe 102, deren Länge und Breite zur Aufnahme eines Teiles des Fadens nach **Fig. 9** bemessen ist.

Die Nadel 120 ist aus federhartem, nichtrostendem Stahl gefertigt. Sie ist mit ihrem Zapfen 124 in der entsprechenden Bohrung mit der unteren Backe 102 verschweißt. Da das federharte Material von Backe 102 und Nadel 120 durch die Schweißung ausgerechnet an der Stelle des größten Biegemomentes auf die Nadel an Festigkeit einbüßt, wurde der Fußquerschnitt der Nadel 120 durch den Kegelstumpf 123 vergrößert. Dadurch wird der Bereich der Schweißnaht weitgehend von Biegebeanspruchung entlastet.

Das freie Ende der Nadel 120 ist zur Spitze 121 geschliffen.

Dicht unterhalb der Basis dieser Spitze 121 hat der zylindrische Nadelschaft den umlaufenden Einstich 122. Zur vorübergehenden Fixierung des Verschlußunterteiles 2 auf der unteren Backe 102 während der Nahtapplikation rastet der Wulst 9 des Verschlußunterteiles 2 - wie in **Fig. 6** dargestellt - in diesen Einstich 122 der Nadel 120 ein.

In den hier gegebenen Darstellungen sind beide entspannten Backen 102 und 103 am Beginn des Arbeitszyklus - entsprechend **Fig. 6** - etwa V-förmig nach außen gebogen, wobei die obere Backe 103 relativ zur unteen Backe 102 nach proximal zurückversetzt bleibt. Durch diese Maßnahme und durch die nach distal geneigte Nadel 120 auf der unteren Backe 102 wird das Aufsuchen einer Durchstichstelle im Inzisionsrand erleichtert.

Es ist aber auch eine Konstruktion realisierbar, bei der die untere Backe 102 gerade ist und bleibt und die obere Backe 103 zur Einhaltung der vorgegebenen gegenseitigen Abstände entsprechend stärker gebogen ist. Dann müssen jedoch die freien Backenlängen entsprechend vergrößert werden, um beim Einzug der oberen Backe 103 in die obere Führungsnut 109 den Bereich ihrer elastischen Verformbarkeit nicht zu überschreiten. Die in den hier vorliegenden Figuren dargestellte Formgebung ist das Ergebnis von FEM-Rechnungen.

Das Einfädeln der Backen 102 und 103 in die Führungsnuten 110 und 109 beim Zusammenbau des Instrumentes - z.B. nach einer Reinigung - wird durch den Hohlkegel 117 in der proximalen Stirnseite des Profilzylinders 101 und durch den Stempel 45 am distalen Ende des Schaftinnenteiles 42 erleichtert. Einerseits stützen sich die Backen 102 und 103 beim Durchschieben durch das Schaftrohr 41 mit ihren zur Instrumentenachse weisenden Flächen auf den entsprechenden Flächen des Stempels 45 ab. Andererseits liegen sie mit ihren Längskanten an der Innenwand des Schaftrohres 41 an, so daß sie entgegen ihrer Federkraft gerade gestreckt bleiben und daher zentriert durch den Hohlkegel 117 leicht in die Führungsnuten 109 und 110 im Profilzylinder 101 einspuren.

Nach der Beschreibung der Komponenten des Applikatorkopfes 100 werden nun ihre Positionen zueinander im Verlaufe eines ganzen Programmzyklus kurz vorgestellt. Auf die Wiederholung der bereits gegebenen Beschreibung von Bedienungsabläufen am Applikatorgriff 50 wird dabei weitgehend verzichtet.

Denkt man sich aus der **Fig. 6** den Faden mit seinen Verschlußteilen 2 und 3 entfernt, so hat man die Ausgangsstellung der Backen 102 und 103 relativ zueinander und zum Profilzylinder 101. Auf die Nadel 120 werden mittels des Übergabehalters 30 nach **Fig. 2** die in ihm gefaßten Verschlußteile 2 und 3 am Faden aufgehängt.

Der Profilzylinder 101 und die obere Backe 103 werden anschließend nach distal vorgeschoben, bis die in der **Fig. 3** dargestellte Anordnung erreicht ist. In Längsrichtung ändert sich die relative Lage der Backen 102 und 103 nun nicht mehr. Nach weiterem distalen Vorschub des Profilzylinders 101 über die Backen 102 und 103 gelangt der Applikatorkopf 100 in die in **Fig. 8** gezeigte Stellung. Folgende Einzelvorgänge sind dabei abgelaufen:
Während die distalen Enden der Backen 102 und 103 sich annähern, spurt das Verschlußoberteil 3 zunächst in den Druchbruch 105 der oberen Backe 103 ein. Danach wird durch die Kraft der sich immer weiter annähernden Backen 102 und 103 sowohl das Verschlußunterteil 2 auf die Nadel 120 gedrückt und durch den Wulst 9 in dem Einstich 122 gegen späteres unbeabsichtigtes Abgleiten gesichert als auch der Durchbruch 105 in der oberen Backe 103 unter seitlichem Aufspreizen der Arme 104 über die Rippen 19 des Verschlußoberteiles 3 geklippt. Damit ist auch das Verschlußoberteil 3 in der oberen Backe 103 fixiert. Ein Zusammendrücken und Verriegeln der Verschlußteile 2 und 3 ist bei diesem Beladevorgang nicht möglich, da die Teile durch den Übergabehalter 30 auf Abstand gehalten werden.

Das Instrument ist in der in **Fig. 8** gezeigten Stellung durch die Programmechanik im Griffkopf 51 blockiert. Der Übergabehalter 30 nach **Fig. 2** und **3** wird nach distal von den Verschlußteilen 2 und 3 abgezogen, so daß der Faden, wie in der **Fig. 8** dargestellt, geschlossen aber nicht verriegelt für eine Applikation zur Verfügung steht.

Der so geladene Applikator wird entweder bis zu seinem Einsatz auf dem Instrumententisch abgelegt oder sofort dem Operateur übergeben, der den Applikatorkopf 100 durch eine Trokarhülse in den Operationssitus einführt.

Wird nun im Operationsfeld, wie im vorhergehenden Abschnitt beschrieben, die Bockierung des Instrumentes über die Betätigung des Federhebels 57 aufgehoben, so kann die in der **Fig. 6** gezeigten Arbeitsstellung der Backen 102 und 103 mit dem eingelegten Faden angefahren werden.

Die Spitze 121 der Nadel 120 wird unter einen Geweberand positioniert. Durch distales Vorschieben der oberen Backe 103 und des Profilzylinders 101 nimmt der Applikatorkopf 100 die in der **Fig. 8** gezeigte Stellung ein, wobei das Gewebe durchstochen und auf das Verschlußunterteil 2 aufgeladen wird.

Das derart gefaßte Gewebe wird dem zweiten Wundrand angenähert. Der Profilzylinder 101 sowie die obere Backe 103 werden erneut zurückgezogen, so daß in der in **Fig. 6** gezeigten Stellung des Applikatorkopfes 100 der zweite Wundrand unterfahren werden kann. Nach Durchstechen und Aufladen des zweiten Geweberandes nehmen Applikatorkopf 100 und Faden mit seinen Verschlußteilen 2 und 3 wieder die in **Fig. 8** dargestellten Positionen ein: Die Fadenschlinge ist geschlossen, aber der Verschluß noch nicht verriegelt.

Dazu wird durch Betätigen des Federhebels 57 sein Zapfen 58 in die Verriegelungskulissenbahn 71 der Programmechanik am Applikatorgriff 50 gedrückt, um jetzt durch weiteres Anziehen des Finger-Griffteiles 53 den Profilzylinder 101 weiter nach distal über die Backen 102 und 103 und über die Fadenverschlußteile 2 und 3 schieben zu können.

Dabei laufen die Abschrägung 17 an der oberen Stirnseite des Verschlußoberteiles 3 auf die Abschrägungen 112 der distalen oberen Kante des Durchbruches 125 bzw. auf die Abschrägungen 111 der distalen oberen Kanten der Längsrippen 118 über der unteren Führungsnut 110 auf.

Durch weiteren distalen Vorschub des Profilzylinders 101 gleiten die Verschlußteile 2 und 3 des Fadens 1 axial weiter ineinander. Dabei wird gleichzeitig das Verschlußoberteil durch das Abgleiten der unteren Abschrägung 21 seiner Rippen 19 auf den oberen Abschrägungen 106a im Durchbruch 105 der oberen Backe 103 aus seiner Klemmung gelöst. Ebenso wird durch Abheben des Verschlußunterteiles 2 von der unteren Backe 102 der Wulst 9 am oberen Rand der Bohrung 7 im Verschlußunterteil 2 nach oben aus dem Einstich 122 hinausgedrückt. Die so erreichten Positionen des verriegelten Fadenverschlusses und Applikatorkopfes sind in der **Fig. 9** dargestellt.

Durch Freigabe des Finger-Griffteiles 53 bis zu seinem Endanschlag wird entsprechend **Fig. 10** die Ausgangsanordnung des Applikatorkopfes 100 erreicht, in der der Verschluß der fertigen, die Geweberänder 126 umfassenden Einzelknopfnaht leicht von der Nadel 120 abgleitet.

Der in der **Fig. 10** dargestellte - jetzt allerdings vom Faden und seinen Verschlußteilen 2 und 3 befreite - Applikatorkopf 100 könnte anschließend einfach durch die Trokarhülse aus dem Operationsfeld extrahiert werden. Die Backen 102 und 103 werden dann in der Trokarhülse gegen ihre Federkraft zusammengedrückt. Bei diesem Vorgehen besteht jedoch die Gefahr einer Beschädigung der weit vor der distalen Stirnseite des Profilzylinders 101 frei und ungeschützt liegenden Nadel 120.

Um diese mögliche Nadelbeschädigung zu vermeiden, zieht der Operateur nach der Freigabe der gelegten Einzelknopfnaht das Finger-Griffteil 53 noch einmal so weit an, bis er wegen des Anlaufens des Zapfens 58 des Federhebel 57 an die Schwelle 65 in der Beladekulissenbahn 64 einen ausgeprägten Druckpunkt wahrnimmt. Dem Druckpunkt entspricht dann etwa die in **Fig. 8** - aber ohne Faden - dargestellte Anordnung der Backen 102 und 103 relativ zueinander und zum Profilzylinder 101, so daß die Nadel 120 und ihre Spitze 121 gut geschützt durch die Trokarhülse gleiten können.

Nach dem Auszug des Applikatorkopfes 100 aus der Trokarhülse gibt der Operateur das Finger-Griffteil 53 wieder frei, die Backen 102 und 103 spreizen V-förmig auseinander - entsprechend der **Fig. 6** und **10**, jedoch ohne Faden -, und der Applikator kann von der OP-Schwester zur Beladung mit dem nächsten Faden übernommen werden.

### Zerlegen und Zusammenbau

Die Konstruktion des vorgeschlagenen Nähinstruments wurde unter besonderer Berücksichtigung der Möglichkeit zur umfassenden Reinigung und Sterilisation des Instruments durchgeführt. Daher ist das Instrument vollständig und ohne Werkzeuge in seine Einzelteile zerlegbar. Nicht zerlegbare Gelenke sind nicht vorhanden. Nach der Demontage des Instrumentes sind das Lumen des Schaftrohres 41 und des angeschweißten Profilzylinders 101 spülbar und eventuell mit einer entsprechenden Rundbürste mechanisch zu säubern.

### Bezugzeichenliste

### Faden mit Verschlußteilen

- 1: Fadenabschnitt
- 2: Verschlußunterteil
- 3: Verschlußoberteil
- 4: Platte
- 5: Hohlzylinder
- 6: Übergangskegel
- 7: Bohrung des Hohlzylinders 5
- 8: Bohrung in der Platte 4
- 9: Wulst in der Bohrung 7
- 10: abgeschrägter freier Rand des Hohlzylinders 5
- 11: Aussparungen im Außenmantel des Hohlzylinders 5
- 12: Abschrägungen an der Unterseite der Platte 4
- 13: vordere Rippe am Verschlußoberteil 3
- 14: hintere Rippen am Verschlußoberteil 3
- 15: Vierkantrohr
- 16: Bohrung des Vierkantrohres 15
- 17: Abschrägung der oberen Stirnfläche des Vierkantrohres 15
- 18: Abschrägung der oberen Stirnseite des Vierkantrohres 15
- 19: Rippen an den Seitenflächen des Vierkantrohres 15
- 20: Nuten für Übergabehalter
- 21: untere Abschrägung der Rippen 19
- 22: obere Abschrägung der Rippen 19
- 23: Nasen in der Bohrung 16
- 24: Kanäle
- 25: untere Stirnfläche des Vierkantrohres 15
- 26: obere Fläche der Platte 4

### Übergabehalter

- 30: Übergabehalter
- 31a: gabelförmiger Kopf
- 31b: flexibler Griff
- 32: Aussparung
- 33: obere Aussparung für Verschlußoberteil 3
- 34: mittlere Aussparung für den Hohlzylinder 5 des Verschlußunterteiles 2
- 35: untere Aussparung zur Aufnahme und Führung der Platte 4 des Verschlußunterteiles 2
- 36: Rippen in der oberen Aussparung 33
- 37: Rippen in der mittleren Aussparung 34
- 38: Auflagefläche für die Platte 4 des Verschlußunterteiles 2
- 39: Auflagefläche für das Verschlußoberteil 3

### Applikatorschaft

- 40: Applikatorschaft
- 41: Schaftrohr
- 42: Schaftinnenteil
- 43: gerade Verlängerung der oberen Backe 103
- 44: Längsnut im Schaftinnenteil 42
- 45: Stempel am distalen Ende des Schaftinnenteiles 42
- 46: Abplattung des Schaftinnenteiles proximal des Stempels 45
- 47: Längsnut in der Abplattung 46

### Applikatorgriff

- 50: Applikatorgriff, komplett
- 51: Griffkopf
- 52: Handteller-Griffteil
- 53: Finger-Griffteil
- 54: Kreuzkopf
- 55: Querschlitze in der Hülse 82
- 56: Winkelstück
- 56a: Gleitfläche des Winkelstückes 56
- 56b: Anschlagfläche des Winkelstücks 56
- 56c: Bohrung im Winkelstück 56
- 57: Federhebel
- 58: Zapfen am Federhebel 57
- 59: federnde Zunge am Federhebel 57
- 60: Druckplatte am Federhebel 57
- 61: Abkantung am Federhebel 57
- 62: Nase am Kreuzkopf 54
- 63: abgeschrägte Rippe
- 64: Beladekulissenbahn
- 64a: waagerechte Ebene der Beladekulissenbahn 64
- 64b: Rampe der Beladekulissenbahn 64
- 65: Schwelle in der Beladekulissenbahn 64
- 66: Kante in der Beladekulissenbahn 64
- 67: Vertiefung in der Beladekulissenbahn 64
- 68: angefaste Rippe zwischen der Belade- und Arbeitskulissenbahn
- 69: Arbeitskulissenbahn
- 70: angefaste Rippe zwischen der Arbeits- und Verriegelungskulissenbahn
- 71: Verriegelungskulissenbahn
- 71a: Vertiefung in der Verriegelungskulissenbahn 71
- 71b: schräge Rampe der Verriegelungskulissenbahn 71
- 72: obere Kante in der Verriegelungskulissenbahn 71
- 73: Rücklaufkulissenbahn
- 74: Rändelschraube für Federhebel 57
- 75: Rändelschraube in der Verlängerung 87 des Schaftinnenteiles 42
- 76: Rändelschraube am Kreuzkopf 54
- 77: Rändelschraube für Finger-Griffteil 53
- 78: Querhaupt am proximalen Ende der geraden Verlängerung 43 der oberen Backe 103
- 79: distale Anlageflächen des Querhauptes 78
- 80: Aussparung in der Verlängerung 87 des Schaftinnenteiles 42
- 81: distale Anschläge in der Aussparung 80
- 82: quergeschlitzte Hülse
- 83: Anlagefläche für das Querhaupt 78 und die quergeschlitzte Hülse 82 in der Bohrung 93
- 84: Druckfeder in der Bohrung 93
- 85: gerändelte Hohlschraube
- 86: Stift im Winkelstück 56
- 87: Verlängerung des Schaftinnenteiles 42
- 88: Gabel am Finger-Griffteil 53
- 89: Gabelwange mit Gewindebohrung
- 90: Gabelwange mit glatter Bohrung
- 91: Langlöcher in den Gabelwangen
- 92: Querbohrung im Winkelstück 56 zur Lagerung des Finger-Griffteiles 53
- 93: Längsbohrung im Winkelstück 56
- 94: Schwenkwinkel des Finger-Griffteiles 53
- 95: Längsgeteilte Montagehülse
- 97: Rückstellfeder
- 98: Gelenkscheiben an den Enden der Rückstellfeder 97
- 99: Gelenktaschen in den Griffteilen 52 und 53

### Applikatorkopf

- 100: Applikatorkopf, komplett
- 101: Profilzylinder
- 102: untere Backe mit der Nadel 120
- 103: obere Backe mit dem Durchbruch 105
- 104: Arme der geschlitzten oberen Backe 103
- 105: viereckiger Durchbruch in der oberen Backe 103
- 106a: obere Schräge im Durchbruch 105
- 106b: untere Schräge im Durchbruch 105
- 107: Längsrippe auf der unteren Backe 102
- 108: Längsnut in der Längsrippe 107
- 109: obere Führungsnut für die obere Backe 103
- 110: untere Führungsnut für die untere Backe 102
- 111: Abschrägung der Rippen 118
- 112: Abschrägung der distalen oberen Kante des Rechteckdurchbruches 125 im Profilzylinder 101
- 113: Längsnut über dem Rechteckdurchbruch 125
- 114: Querschlitz durch die Achse des distalen Endes des Profilzylinders 101
- 115: Gabelunterteil des Profilzylinders 101
- 116: Gabeloberteil des Profilzylinders 101
- 117: Hohlkegel im proximalen Ende des Profilzylinders 101
- 118: Längsrippen über der unteren Führungsnut 110
- 119: Längsrippen unter der oberen Führungsnut 109
- 120: Nadel auf der unteren Backe 102
- 121: Spitze der Nadel 120
- 122: Einstich im Nadelschaft
- 123: Kegelstumpf zur Vergrößerung der Nadelbasis
- 124: Zapfen des Nadelfußes
- 125: achsfluchtender Rechteckdurchbruch im Profilzylinder 101
- 126: Geweberänder

## Patentansprüche

1. Faden zum Anlegen einer chirurgischen Naht bestehend aus
a) einem Fadenabschnitt (1) aus einem biegsamen Material, der ein erstes und ein zweites Ende aufweist;
b) einem Verschlußunterteil (2) am ersten Ende des Fadenabschnitts (1), das
- einen Zylinder (5) aufweist, der
- mittelbar oder unmittelbar über eines seiner Enden mit dem Fadenabschnitt (1) verbunden ist und dessen freies, nicht mit dem Fadenabschnitt (1) verbundenes Ende vom Fadenabschnitt (1) wegweist und
- an seinem freien Ende in einen abgeschrägten Rand (10) ausläuft,
c) einem Verschlußoberteil (3) am zweiten Ende des Fadenabschnitts (1), das aus einem rohrförmigen Teil (15) besteht, in das der Zylinder (5) einsetzbar ist,
d) Mitteln (11, 23), mit deren Hilfe sich das Verschlußoberteil (3) mit dem Verschlußunterteil (2) verriegeln läßt,
dadurch gekennzeichnet, daß
e) der Zylinder (5) ein Hohlzylinder mit einer durchgehenden Bohrung (7) ist, wobei
- die Bohrung (7) des Hohlzylinders (5) in der Weise gestaltet ist, daß eine chirurgische Nadel (120) einsetzbar ist, so daß
- die Spitze (121) der chirurgischen Nadel aus dem freien Ende des Hohlzylinders (5) herausragt und
- zusammen mit dem abgeschrägten Rand (10) eine glatte Übergangsfläche ausbildet und
f) das rohrförmige Teil (15) des Verschlußoberteils (3) in Form eines Vierkantrohres gestaltet ist.

2. Faden nach Anspruch 1 mit dem Verschlußunterteil (2), bei dem sich der Hohlzylinder (5) auf einer mit dem Fadenabschnitt (1) verbundenen, durchbrochenen Platte (4) erhebt, die eine kegelstumpfförmige, durchgehende Bohrung (8) mit einer kleineren und einer größeren Öffnung aufweist, deren kleinere Öffnung dem Hohlzylinder (5) zugewandt ist und mit dessen Bohrung (7) fluchtet und deren größere Öffnung dem Hohlzylinder (5) abgewandt ist.

3. Faden nach Anspruch 1 oder 2, bei dem die Mittel, mit deren Hilfe sich das Verschlußoberteil (3) mit dem Verschlußunterteil (2) verriegeln läßt, aus mindestens einer Nase (23) im Innenraum des rohrförmigen Teils und mindestens einer Aussparung (11) am Hohlzylinder (5) bestehen, wobei die Nase (23) in die Aussparung (11) eingreift.

4. Kombination bestehend aus einem Übergabehalter (30) zur Handhabung und dem Faden nach einem der Ansprüche 1 bis 3 mit dem Übergabehalter bestehend aus
a) einem gabelförmigen Kopf (31a), der Aussparungen (32) zur Aufnahme sowohl des Verschlußunterteils (2) als auch des Verschlußoberteils (3) aufweist, und
b) einem Griff.

5. Kombination bestehend aus einem chirurgischen Nähinstrument zum Anlegen einer chirurgischen Naht und dem Faden nach einem der Ansprüche 1 bis 3 dem chirurgischen Nähinstrument bestehend aus
a) einem Applikatorschaft (40), der
- ein distales, in den Körper eines Patienten einzuführendes Ende und ein proximales Ende aufweist und
- aus einem Schaftinnenteil (42) und einem gegenüber dem Schaftinnenteil (42) axial verschiebbaren äußeren Schaftrohr (41) besteht,
b) zwei einander gegenüberliegenden Backen (102, 103) am distalen Ende des Applikatorschafts (40), die aufeinander zu und voneinander weg bewegt werden können,
c) einem mehrteiligen Griffstück (52, 53) am proximalen Ende des Applikatorschafts (40) zur Betätigung der Backen (102, 103), von dem ein Teil (52) mit dem Schaftinnenteil (42) und ein weiteres Teil (53) mit dem äußeren Schaftrohr (41) verbunden ist,
wobei
d) die Backen (102, 103) aus einem federelastischen Material bestehen und ohne Krafteinwirkung eine V-förmige Stellung zueinander einnehmen und
e) eine der beiden Backen (102) an ihrem distalen Ende mit einer Nadel (120) versehen ist, die in eine Spitze (121) ausläuft, und deren Länge maximal dem Innendurchmesser des äußeren Schaftrohrs (41) entspricht.

6. Kombination nach Anspruch 5 mit Mitteln, die
- ein distales Vorschieben der mit der Nadel (120) versehenen Backe (102) relativ zu der ihr gegenüberliegenden Backe (103) und/oder
- ein proximales Zurückziehen der nicht mit der Nadel (120) versehenen Backe (103) relativ zu der ihr gegenüberliegenden Backe (102) ermöglichen.

## Claims

1. Thread for making a surgical suture, comprising:
a) a thread portion (1), which is formed from a flexible material and has a first end and a second end,
b) a lower closure part (2) on the first end of the thread portion (1), which
- has a cylinder (5), which
- is indirectly or directly connected to the thread portion (1) via one of its ends, and the free end of which cylinder, not connected to the thread portion (1), points away from the thread portion (1), and
- extends at its free end into a sloping edge (10),
c) an upper closure part (3) on the second end of the thread portion (1), which comprises a tubular part (15), into which the cylinder (5) is insertable, and
d) means (11, 23), whereby the upper closure part (3) can lock with the lower closure part (2),
characterised in that
e) the cylinder (5) is a hollow cylinder with a through-bore (7),
- the bore (7) of the hollow cylinder (5) being configured in such a manner that a surgical needle (120) is insertable, so that
- the point (121) of the surgical needle protrudes from the free end of the hollow cylinder (5) and
- forms, together with the sloping edge (10), a smooth transitional surface, and
f) the tubular part (15) of the upper closure part (3) is in the form of a square tube.

2. Thread according to claim 1 having the lower closure part (2), wherein the hollow cylinder (5) is raised on a discontinuous plate (4), which is connected to the thread portion (1) and has a frustoconical, through-bore (8) provided with a smaller opening and a larger opening, the smaller opening of which faces the hollow cylinder (5) and is in alignment with the bore (7) thereof, and the greater opening of which is remote from the hollow cylinder (5).

3. Thread according to claim 1 or 2, wherein the means, whereby the upper closure part (3) can lock with the lower closure part (2), comprise at least one projection (23) in the interior of the tubular part and at least one recess (11) on the hollow cylinder (5), the projection (23) engaging in the recess (11).

4. Combination, comprising a transfer holder (30) for handling purposes and the thread according to one of claims 1 to 3, with the transfer holder comprising
a) a bifurcated head (31a), which has recesses (32) for accommodating both the lower closure part (2) and the upper closure part (3), and
b) a handle.

5. Combination, comprising a surgical suturing instrument for making a surgical suture and the thread according to one of claims 1 to 3, the surgical suturing instrument comprising
a) an applicator shaft (40), which
- has a distal end, which is to be inserted into the body of a patient, and a proximal end, and
- comprises an inner shaft part (42) and an outer shaft tube (41) which is axially displaceable relative to the internal shaft part (42),
b) two jaws (102, 103) on the distal end of the applicator shaft (40), which jaws are situated opposite each other and can be moved towards each other and away from each other,
c) a multi-part gripping part (52, 53) on the proximal end of the applicator shaft (40) for actuating the jaws (102, 103), one part (52) of which is connected to the internal shaft part (42), and an additional part (53) is connected to the outer shaft tube (41),
d) the jaws (102, 103) being formed from a resilient material and assuming, without the action of force, a V-shaped position relative to each other, and
e) one of the two jaws (102) being provided at its distal end with a needle (120), which extends into a point (121), and the maximum length of which corresponds to the internal diameter of the outer shaft tube (41).

6. Combination according to claim 5, having means which render possible
- a distal advancing movement of the jaw (102), which is provided with the needle (120), relative to the jaw (103), which is situated opposite thereto, and/or
- a proximal withdrawing movement of the jaw (103), which is not provided with the needle (120), relative to the jaw (102), which is situated opposite thereto.

## Revendications

1. Fil pour application d'une suture chirurgicale, constitué :
a) d'un tronçon de fil (1) constitué d'un matériau flexible, présentant une première et une deuxième extrémité ;
b) d'une partie inférieure de fermeture (2), située à la première extrémité du tronçon de fil (1), qui
- présente un cylindre (5), qui
- est relié, indirectement ou directement, au tronçon de fil (1) par l'une de ses extrémités, et dont l'extrémité libre, non reliée au tronçon de fil (1), est tournée de façon à s'éloigner du tronçon de fil (1), et
- passe, à son extrémité libre, dans un bord (10) biseauté,
c) une partie supérieure de fermeture (3) située à la deuxième extrémité du tronçon de fil (1), qui est constituée d'une partie (15) tubulaire, dans laquelle le cylindre (5) peut être inséré,
d) des moyens (11, 23) à l'aide desquels la partie supérieure de fermeture (3) peut être verrouillée à la partie inférieure de fermeture (2),
caractérisé en ce que
e) le cylindre (5) est un cylindre creux ayant un perçage (7) traversant continu,
- le perçage (7) du cylindre creux (5) étant configuré de manière à ce qu'une aiguille chirurgicale (120) puisse être insérée, de manière que
- la pointe (121) de l'aiguille chirurgicale sorte de l'extrémité libre du cylindre creux (5), et
- et conjointement avec le bord (10) chanfreiné, une surface de transition est réalisée, et
f) la partie (15) tubulaire de la partie supérieure de fermeture (3) étant configurée sous la forme d'un tube à section carrée.

2. Fil selon la revendication 1, avec la partie de fermeture (2),
pour lequel
le cylindre creux (5) monte depuis une plaque (3) dotée de perforations, reliée au tronçon de fil (1), plaque présentant un perçage (8) traversant continu, à forme tronconique, ayant une petite ouverture et une grande ouverture, perçage dont la petite ouverture est tournée vers le cylindre creux (5) et est aligné avec son perçage (7), et dont la grande ouverture est opposée au cylindre creux (5).

3. Fil selon la revendication 1 ou 2,
pour lequel
les moyens, à l'aide desquels la partie supérieure de fermeture (3) peut être verrouillée dans la partie inférieure de fermeture (2), sont constitués d'au moins un ergot (23) placé dans l'espace intérieur de la partie à forme tubulaire et au moins un évidement (11) ménagé sur le cylindre creux (5), l'ergot (23) s'engageant dans l'évidement (11).

4. Combinaison, constituée d'un support de transfert (30) destiné à la manipulation du fil selon l'une des revendications 1 à 3, le support de transfert étant constitué :
a) d'une tête fourchue (31a), présentant des évidements (32) destinés à recevoir tant la partie inférieure de fermeture (2) qu'également la partie supérieure de fermeture (3), et
b) une poignée.

5. Combinaison, constituée d'un instrument de suture chirurgicale, pour appliquer une suture chirurgicale, et du fil selon l'une des revendications 1 à 3, l'instrument de suture chirurgicale étant constitué :
a) d'une tige d'applicateur (40), qui
- présente une extrémité distale à insérer dans le corps d'un patient et une extrémité proximale, et
- d'une partie intérieure de tige (42) et d'un tube de tige extérieur (41) déplaçable axialement par rapport à la partie intérieure de tige (42),
b) avec deux mâchoires (102, 103) opposées l'une à l'autre, à l'extrémité distale de la tige d'applicateur (40), qui peuvent être rapprochées et espacées l'une de l'autre,
c) d'un élément formant poignée (52, 53) en plusieurs parties, à l'extrémité proximale de la tige d'applicateur (40), pour actionner les mâchoires (102, 103), dont une première partie (52) est reliée à la partie intérieure de tige (42) et une autre partie (53) est reliée au tube de tige extérieur (41), où
d) les mâchoires (102, 103) sont constituées d'un matériau ayant l'élasticité d'un ressort et peuvent prendre l'une par rapport à l'autre, en l'absence d'effort, une position en forme de V, et
- l'une des deux mâchoires (102) étant dotée, sur son extrémité distale, d'une aiguille (120) qui évolue en une pointe (121) et dont la longueur correspond au maximum au diamètre intérieur du tube de tige extérieur (41).

6. Combinaison selon la revendication 5, avec des moyens qui :
- permettent un avancement distal de la mâchoire (102) dotée de l'aiguille (120) par rapport à la mâchoire (103) placée en regard d'elle et/ou
- un retrait proximal de la mâchoire (103) non dotée de l'aiguille (120) par rapport à la mâchoire (102) placée en regard d'elle.
